Europäisches Patentamt

**European Patent Office**

Office européen des brevets

(19)

(11) Numéro de publication : **0 014 003 B1**

(12)                    **FASCICULE DE BREVET EUROPÉEN**

(45) Date de publication du fascicule du brevet :
**15.12.82**

(21) Numéro de dépôt : **80200001.8**

(22) Date de dépôt : **02.01.80**

(51) Int. Cl.³ : **C 12 N 11/00, C 12 P 19/24, C 12 P 7/58**

(54) **Granules complexes contenant des substances protéiniques actives et procédés pour leur fabrication, leur utilisation, et leur régénération.**

(30) Priorité : **12.01.79 FR 7901054**

(43) Date de publication de la demande :
**06.08.80 (Bulletin 80/16)**

(45) Mention de la délivrance du brevet :
**15.12.82 Bulletin 82/50**

(84) Etats contractants désignés :
**AT BE CH DE FR GB IT LU NL SE**

(56) Documents cités :
**FR A 2 035 774**
**FR A 2 218 344**
**FR A 2 403 384**
**US A 4 141 857**
**CHEMICAL ABSTRACTS, vol. 72, no. 24, 15 june 1970, page 191, ref. 124114w Columbus, Ohio, US R.A. SANDLER et al. : « Secondary structure of a titanium sponge prepared by the magnesiothermic reduction of lower chlorides »**
**CHEMICAL ABSTRACTS, vol. 90, no. 15, 9 avril 1979, page 260, ref. 117193q Columbus, Ohio, US V.D. SEMICHAEVSKII : « Immobilization of cellulases and pectinases of Coriolus versicolor (L. ex FR.) Quel. on solid carriers using serum albumin.**

(73) Titulaire : **SOLVAY & Cie (Société Anonyme)**
**Rue du Prince Albert, 33**
**B-1050 Bruxelles (BE)**

(72) Inventeur : **Coppens, Guillaume**
**Avenue Louis Jasmin, 89**
**B-1150 Bruxelles (BE)**
Inventeur : **Pêtre, Jean**
**Rue Charles Lemaire 26**
**B-1160 Bruxelles (BE)**

(74) Mandataire : **Elschen, Roland**
**Solvay & Cie Département de la Propriété Industrielle**
**Rue de Ransbeek 310**
**B-1120 Bruxelles (BE)**

Jouve, 18, rue St-Denis, 75001 Paris, France

Granules complexes contenant des substances protéiniques actives et procédés pour leur fabrication, leur utilisation, et leur régénération

La présente invention concerne des granules complexes insolubles dans l'eau, contenant des substances protéiniques actives telles que des enzymes qui peuvent être utilisées comme catalyseurs. L'invention concerne également un procédé pour la fabrication de tels granules, leur utilisation pour la catalyse de réactions chimiques, et leur régénération après désactivation.

L'utilisation en tant que catalyseurs de substances protéiniques actives présente un intérêt croissant. Il en est ainsi des enzymes dont le pouvoir catalytique est mis à profit notamment pour effectuer certaines réactions chimiques nécessitant une sélectivité élevée. Etant donné le coût de ces substances, il est essentiel, pour des raisons économiques, de les utiliser dans des procédés continus. Or, les enzymes sont des produits peu stables et solubles dans l'eau, ce qui rend leur séparation, et donc leur récupération, très malaisée.

Diverses méthodes ont été proposées pour immobiliser des substances protéiniques actives telles que les enzymes. On a ainsi proposé de coréticuler ces substances protéiniques actives avec des substances protéiniques inactives (brevet français 68 146 205 (FR-A-16 04 982) déposé le 29 mars 1968 au nom du Centre National de la Recherche Scientifique et les brevets d'addition 69/01 451 (FR-A-2 028 702) déposé le 24 janvier 1969, 69/07 897 (FR-A-2 035 774) déposé le 19 mars 1969 et 70/33 059 (FR-A-2 107 801) déposé le 11 septembre 1970 au nom de l'Agence Nationale de Valorisation de la Recherche). Le produit ainsi obtenu, bien qu'insoluble dans l'eau, n'est cependant pas adapté à une utilisation industrielle. Il se présente en effet sous une forme non rigide et lorsqu'il est mis en œuvre dans les réacteurs, on observe un écrasement du produit entraînant des pertes de charge et même des bouchages complets des réacteurs.

Pour pallier ces inconvénients, on a proposé d'effectuer cette coréticulation en présente de petites particules de ferrites (G. Gellf et J. Boudrant, Biochimica et Biophysica Acta, 1974, 334, p. 467-470). Dans ce cas, le produit de coréticulation de la substance protéinique active avec la substance protéinique inactive est fixé par simple contact sur la surface externe des particules de ferrite qu'il enrobe, de sorte que, lors de l'utilisation industrielle, on observe une forte attrition entraînant l'arrachement du produit de coréticulation de son support, ce qui conduit aux mêmes inconvénients que la technique antérieure.

Par ailleurs, on a déjà proposé de fixer des enzymes sur des supports poreux. Plus particulièrement, on décrit dans le brevet Etats-Unis US-A 3 666 627 déposé le 14.10.68 et cédé à CORNING GLASS WORKS, un procédé dans lequel l'enzyme est fixée par adsorption sur le support inorganique, tel que le verre poreux, qui est au préalable traité avec un substrat compatible avec l'enzyme. Les complexes ainsi obtenus ne donnent toutefois pas satisfaction sur le plan industriel car ils perdent leur activité enzymatique suite à une dissolution et désorption rapide dans les milieux réactionnels habituellement utilisés du substrat et de l'enzyme.

Par après, on a proposé dans le brevet belge BE-A-803 275 déposé le 6.8.1973 au nom de CORNING GLASS WORKS, un procédé pour immobiliser des enzymes à l'intérieur des pores et sur la surface d'un support minéral poreux dont le diamètre des pores est inférieur à 1 000 Å. Ce procédé nécessite l'emploi en alternance de solvants non aqueux et de solutions aqueuses étant donné l'incompatibilité entre les supports ayant les caractéristiques préconisées, les enzymes et les agents de réticulation solubles dans l'eau. Ce procédé implique par conséquent une série d'opérations de séparation et de séchage qui le rendent peu attrayant d'un point de vue industriel. En outre, les risques de dénaturation des enzymes résultant de l'emploi de solvants est loin d'être négligeable, de sorte que pour chaque enzyme il faut rechercher un solvant convenable.

Enfin, on a également proposé d'effectuer l'immobilisation d'enzymes sur divers autres supports inorganiques et notamment des poudres de titane (V.D. SEMICHAEVSKII, Ukr. Bot. Zh., 1978, 35 (6), p. 634-639). Ces poudres sont constituées de particules de dimensions inférieures à 250 µm. Elles présentent les mêmes inconvénients que les autres supports d'enzymes connus.

La présente invention vise à pallier les inconvénients liés à ces différentes techniques et permet l'obtention de substances protéiniques actives, telles que les enzymes, immobilisées sous forme de granules insolubles dans l'eau, rigides et résistants à l'attrition. Ces granules conviennent très bien à un usage industriel et présentent en outre une activité excellente.

La présente invention concerne à cet effet des granules complexes contenant des substances protéiniques actives fixées sur un support solide minéral poreux insoluble dans l'eau qui est constitué de granules comportant à l'intérieur de leur structure des cavités dont la majorité ont des dimensions moyennes comprises entre 5 et 200 µm.

Par support insoluble dans l'eau, on entend désigner tous les supports dont la solubilité dans l'eau est inférieure à 0,1 % et de préférence inférieure à 0,01 % en poids à la température ordinaire. Les meilleurs résultats ont été obtenus lorsque les supports ont une solubilité dans l'eau inférieure à 0,001 % à la température ordinaire.

Des supports minéraux de natures diverses peuvent être utilisés tels que de la pierre ponce et des briques pilées d'origines et de compositions diverses. De bons résultats ont été obtenus avec des granules de métaux poreux. Parmi ceux-ci, on préfère les métaux réfractaires et plus particulièrement le titane et le zirconium. Les meilleurs résultats ont été obtenus avec des granules de titane dont la porosité a été obtenue par réduction de sels. Pareil titane spongieux peut être obtenu par réduction du tétrachlorure de titane selon les techniques classiques de la métallurgie du titane telles que décrites par exemple dans l'ouvrage Reine und angewandte Metallkunde in Einzeldarstellungen, Band 21, U. Zwicker, Titan und Titanlegierungen, 1974, Springer-Verlag.

La répartition des dimensions des cavités présentes dans les granules utilisés comme support est en général peu étalée. De préférence, au moins 70 %, du volume total des cavités est constitué de cavités ayant des dimensions comprises entre 5 et 200 μm. Les meilleurs résultats ont été obtenus lorsque 85 % au moins de volume total des cavités est constitué de pareils pores.

Le plus souvent, les granules utilisés comme support comportent des cavités ayant des dimensions moyennes comprises entre 20 et 100 μm.

Les granules mis en œuvre comme support peuvent avoir des dimensions variables sans que, d'habitude, leur diamètre moyen soit inférieur à 100 μm. De préférence, on utilise des granules ayant des dimensions comprises entre 0,01 et 2 cm. Les granules peuvent éventuellement être agglomérés soit entre eux soit avec une autre charge inerte appropriée. Ces agglomérats peuvent avoir des formes quelconques telles que celles de pastilles, de tablettes, de comprimés et de plaques de formes géométriques quelconques.

La porosité des granules utilisés comme support est en général comprise entre 0,05 et 0,6 cm³/g et le plus souvent entre 0,1 et 0,5 cm³/g.

La surface spécifique des granules utilisés comme support est très petite, en général inférieure à 1 m²/g. Le plus souvent, les surfaces spécifiques de ces granules sont comprises entre 0,005 et 0,5 m²/g.

Une qualité particulièrement adéquate de titane spongieux est constituée de granules de dimensions moyennes comprises entre 0,1 et 5 mm de diamètre, de poids spécifique apparent mesuré par écoulement libre compris entre 0,5 et 1,5 kg/dm³, ayant un diamètre moyen des pores compris entre 20 et 100 μm, moins de 15 % des pores ayant des dimensions en dehors de ces limites, et une surface spécifique comprise entre 0,01 et 0,25 m²/g. Ce produit est en général constitué à raison d'au moins 95 % et de préférence d'au moins 97 % de son poids de titane.

Les substances protéiniques actives contenues dans les granules complexes selon l'invention peuvent être de natures très diverses. Elles sont en général choisies parmi les virus, les microbes, les antigènes, les anticorps, les enzymes, et les polypeptides. On peut également immobiliser plusieurs protéines actives différentes sur un même support.

Les polypeptides sont en général choisis parmi les hormones polypeptidiques telles que l'insuline, la vasopressine, l'oxytocine, l'angiotonine, la bradykinine, la kallidine, la bacitracine, la tyrocidine et la gramicidine.

L'invention convient particulièrement bien pour immobiliser les enzymes. Celles-ci peuvent être de natures diverses. Des exemples d'enzymes utilisables sont donnés dans l'ouvrage de H.R. Mahler et E.H. Cordes, Biological Chemistry, Harper Int. Edn. 1967. Des enzymes particulièrement adéquates peuvent être choisies parmi la glucose-déhydrogénase, la glutamique-déhydrogénase, l'alcool-déhydrogénase, la glycéraldéhyde-déhydrogénase, les aminoacide-oxydases, la phénylalanine-hydroxylase, l'acétate-kinase, les énolases, l'uréase, la carboxypeptidase, la papaïne, la renine, la chymotrepsine, la lysine-racémase, l'invertase, l'hexokinase, l'aspartase, la cellulase, la 11-β-hydroxylase et la $\Delta^1$ déshydrogénase.

D'excellents résultats ont été obtenus avec des enzymes telles que la glucose-oxydase, la glucose-isomérase, la catalase, les lactases (dont la β-galactosidase), la fumarase, les aminoacylases, la trypsine, la pepsine, les amylases, la pénicilline-acylase, la pénicilline-amidase et les glycosyltransférases.

Les substances protéiniques actives contenues dans les granules complexes selon l'invention peuvent se trouver sous forme de substances protéiniques libres, sous forme de cellules les contenant ce qui est le cas par exemple, des microorganismes ou également sous forme liée par des liaisons chimiques à d'autres composés d'origine naturelle ou synthétique. Lorsque les substances protéiniques actives sont présentes sous forme de cellules, ces dernières peuvent avoir été soumises à un traitement préalable connu en lui-même tel qu'une lyophilisation ou une lyse partielle ou totale.

La proportion de substances protéiniques actives contenues dans les granules complexes selon l'invention peut varier dans de très larges limites. Elle est en général comprise entre 0,001 et 10 % du poids du support.

Les granules complexes selon l'invention peuvent également contenir un ou plusieurs autres constituants liés ou non à la matière protéinique. Parmi ceux-ci, on peut citer notamment des constituants polymériques inactifs tels que des substances d'origine organique (animale ou végétale) ou des substances macromoléculaires de synthèse. Les substances d'origine organique peuvent être constituées de cellules entières, de cellules partiellement ou complètement lysées, de protéines inactives ou de toute autre substance présente dans les cellules ou microorganismes, ainsi que de mélanges de ces divers produits. Les macromolécules de synthèse peuvent être notamment l'alcool polyvinylique, les polysilanes, les polyacrylamides et leurs produits de substitution.

Une classe particulière de macromolécules de synthèse utilisable pour préparer les granules selon l'invention est constituée par des gels mouillables et plus particulièrement par des gels mouillables constitués d'échangeurs d'ions formant des solutions gélifiables. Habituellement ces gels mouillables sont à base de polymères naturels tels que les polysaccharides ou de la gélatine ou de polymères synthétiques tels que les polyacrylamides, traités par des agents de réticulation bifonctionnels, modifiés par l'introduction de groupes fonctionnels. Comme polysaccharides on utilise en général des composés à base d'agar-agar, agarose, amidon, chitosane, dextrane, séphadex, sépharose et de cellulose. Parmi ceux-ci on préfère ceux à base d'aggarose, de sephadex et de cellulose et tout particulièrement préférés sont les composés à base d'agarose.

Les agents de réticulation sont en général des composés organiques porteurs de fonctions multiples identiques ou différentes, telles que des fonctions amines, aldéhydes, cétones, éther-oxydes et plus particulièrement époxydes, sulfonyles et nitro ainsi que des fonctions diverses contenant le groupe $>C=N^-$. Des exemples d'agents de réticulation sont donnés dans les brevets français précités.

Les agents de réticulation sont avantageusement choisis parmi les aldéhydes, les cétones aromatiques et les amines. Les agents de réticulation préférés sont choisis parmi les aldéhydes aliphatiques tels que le glutaraldéhyde, le formaldéhyde, le glyoxal et l'amidon dialdéhyde, les quinones telles que la benzoquinone, les diamines aliphatiques ou aromatiques telles que l'éthylènediamine, la putrescine, l'hexaméthylènediamine et la phénylènediamine, les polyamines telles que la spermine, la spermidine et le chitosane et les époxydes tels que l'épichlorhydrine. Les meilleurs résultats ont été obtenus avec le glutaraldéhyde, éventuellement en association avec les amines et, de préférence, des diamines, la benzoquinone et l'épichlorhydrine.

Les groupes fonctionnels permettant la modification du polymère sont ceux utilisés dans les échangeurs d'ions classiquement utilisés pour la chromatographie des protéines, notamment des groupes diéthyaminoéthyl (DEAE), carboxyméthyl (CM), phospho (P), sulfoéthyl, triethanolamino, triéthylaminoéthyl, benzyl, alkyl et propyl-triméthylammonium.

Les granules complexes selon l'invention peuvent ainsi contenir des mélanges de protéines extraites des biomasses, des albumines telles que l'albumine bovine ou l'ovalbumine, des collagènes tels que la gélatine, de la caséine, de la cellulose, de l'amylose, des polysaccharides et des alginates.

La proportion de constituants polymériques inactifs éventuellement présents dans les granules complexes selon l'invention peut varier dans de très larges limites. Elle est en général comprise entre 0,001 et 100 % du poids du support. La proportion en poids de substances protéiniques actives par rapport aux constituants polymériques inactifs peut également varier dans d'assez larges limites ; elle est en général comprise entre 20 à 1 et 1 à 1 000.

Les granules complexes selon l'invention peuvent contenir, outre le support poreux et éventuellement les constituants polymériques inactifs, des agents de réticulation. Dans ce cas, les granules complexes se présentent sous la forme d'un support imprégné de substances protéiniques actives et éventuellement de constituants polymériques inactifs réticulés partiellement ou totalement selon toutes les combinaisons possibles au moyen de l'agent de réticulation.

Les agents de réticulation sont en général les mêmes que ceux définis ci-avant et ils peuvent être mis en œuvre dans des proportions très variables. En général, on utilise de 0,01 à 100 % en poids d'agent de réticulation par rapport au poids de substances actives et inactives fixées sur le support.

D'autres additifs encore peuvent également être présents dans les granules complexes selon l'invention, mais, en général, en quantités beaucoup plus faibles, ne dépassant pas le plus souvent 10 % du poids de la composition. Parmi ceux-ci, on peut citer les agents régulateurs de pH, les agents pour la « neutralisation » des agents de réticulation, les produits favorisant l'accrochage chimique des substances protéiniques actives sur le support, les agents bactéricides ou fongicides afin de contrôler une éventuelle pollution bactérienne, et les additifs permettant d'améliorer l'activité de la substance protéinique active.

Le choix de ces divers additifs est fait en fonction du mode de préparation des granules complexes, de leur constitution exacte, et de l'usage auquel on les destine.

La quantité totale de substances présentes dans les granules complexes selon l'invention, à l'exception du support, est en général comprise entre 0,01 et 60 % du poids de la composition.

Les granules complexes selon l'invention se présentent sous forme de particules dont les dimensions extérieures sont à peu près identiques à celles des granules du support solide de départ. Le poids spécifique apparent par écoulement libre des compositions est en général compris entre 0,5 et 2,5 kg/dm³. Les granules peuvent éventuellement être agglomérées après imprégnation sous des formes quelconque telles que celles de pastilles, de tablettes, de comprimés ou des plaques de formes géométriques quelconques.

La présente invention concerne également un procédé pour la fabrication des granules complexes.

Le procédé consiste à imprégner le support minéral par les substances protéiniques actives et les autres constituants éventuels et à effectuer une réticulation.

Ainsi, on peut mettre en contact le support, les substances protéiniques actives, les agents de réticulation et les autres constituants éventuels en présence d'un solvant, réticuler et, ensuite, éliminer tout ou partie du solvant.

La nature du solvant n'est pas critique en elle-même. De préférence, on utilise de l'eau. On peut également utiliser des mélanges de solvants. Dans ce cas, on préfère mettre en œuvre des mélanges d'eau et d'un solvant peu volatil tel que le glycérol par exemple. Dans ce cas, le solvant peu volatil est le plus souvent présent à raison de 0,1 à 20 % du poids d'eau.

La mise en contact du support avec le solvant et les autres constituants peut se faire en une ou plusieurs étapes. Lorsqu'on opère en une seule étape, le support poreux est imbibé au moyen d'un mélange contenant le solvant, l'agent de réticulation, et tous les autres constituants. Ce mélange peut se présenter sous forme de solution, de suspension ou d'émulsion, les divers constituants étant mis en œuvre dans les mêmes proportions que celles souhaitées dans la composition finale.

La réticulation est ensuite réalisée selon toute technique connue en elle-même. Une façon de faire avantageuse consiste à abaisser la température du support imbibé à une valeur inférieure à la température de congélation du solvant liquide. Lorsque le solvant contient de l'eau, on refroidit le support imbibé à une température inférieure à 0 °C, et de préférence comprise entre 0 et − 200 °C. Les meilleurs résultats ont été obtenus lorsqu'on refroidit le support imbibé entre − 20 °C et − 200 °C. Ce refroidissement peut se faire selon diverses techniques. On peut ainsi placer le support imbibé dans une enceinte refroidie à la température désirée et ce pendant en général 2 à 10 heures ; on peut également laisser tomber les granules de support imbibé dans un produit liquide qui est refroidi à la température désirée et dont la température de congélation est inférieure à cette température. L'éther froid ou l'azote liquide figurent parmi les produits liquides convenant pour cet usage. Une fois le solvant congelé, on ramène les granules de support imbibé à la température ambiante, et on élimine tout ou partie du solvant liquide selon toute technique connue en elle-même telle que l'évaporation, l'essorage ou la lyophilisation.

Une autre technique convenant pour effectuer la réticulation consiste à éliminer directement le solvant liquide présent dans le support imbibé selon toute technique connue en elle-même telle que l'évaporation par mise sous vide, par balayage au moyen, d'un gaz inerte (air, azote) sec ou par lyophilisation. Dans ce cas, on utilise avantageusement un solvant constitué d'un mélange d'eau avec de petites quantités d'un liquide peu volatil soluble dans l'eau tel que le glycérol.

L'excès des fonctions réactives des agents de réticulation est en général neutralisé après l'opération de réticulation par addition d'un composé susceptible de réagir avec les groupements fonctionnels présents dans les agents de réticulation. Il est évident que la nature de ces agents de « neutralisation » est choisie en fonction du type d'agent de réticulation mis en œuvre. L'eau et les acides aminés conviennent souvent. Ainsi, lorsque l'agent de réticulation est le glutaraldéhyde, on utilise le plus souvent le glycocolle ou des alkane-diamines comme agent « neutralisant ».

On peut ainsi procéder à un ou plusieurs lavages successifs à l'eau ou au moyen d'une solution aqueuse d'un agent de neutralisation adéquat. Pour des raisons économiques l'eau est utilisée le plus fréquemment.

On peut également effectuer la réticulation en deux étapes. Ainsi, on peut mettre le support poreux en contact avec un premier mélange contenant du solvant, de l'agent de réticulation et tout ou partie des constituants autres que les substances protéiniques actives et procéder à la réticulation selon les techniques précitées et, ensuite, dans une seconde étape, mettre le support ainsi imprégné en contact avec un second mélange contenant du solvant, de l'agent de réticulation, les substances protéiniques actives et éventuellement le solde des autres constituants et procéder à une seconde réticulation selon l'une ou l'autre des techniques précitées.

Suivant une variante du procédé de fabrication des granules décrit ci-avant, on réticule d'abord à l'intérieur des cavités une matière non active à laquelle on accroche ensuite par des liens chimiques la substance protéinique active. La matière non active peut être constituée avantageusement d'un échangeur d'ions à base d'une polysaccharide formant des gels solubles à laquelle il est possible d'accrocher une enzyme par des liens ioniques. Pour ce faire, la polysaccharide peut être d'abord dissoute dans le solvant auquel on ajoute ensuite le support minéral poreux. Après imprégnation du support, celui-ci est traité avec un agent de réticulation de façon à fixer la polysaccharide à l'intérieur des pores. La réticulation est achevée par l'introduction de groupements fonctionnels utilisés classiquement pour la chromatographie des protéines, qui peuvent être notamment les groupements diéthylaminoéthyl-(DEAE)-carboxyméthyle (CM⁻), phospho-(P⁻), triéthylaminoéthyl-(TEAE⁻) ou épichlorhydrine triéthanolamine (ECTEOLA⁻). Le complexe résultant de cette réticulation est un produit possédant des propriétés échangeuses d'ions lesquelles sont mises à profit pour l'accrochage des enzymes par simple lien ionique. Les solvants, agents réticulants et techniques de réticulation utilisés dans cette variante sont les mêmes que ceux décrits ci-avant. L'intérêt de cette variante réside dans le fait que l'enzyme, bien que fixée de façon chimique et stable, est récupérable, qu'elle permet de conserver le support et la matière non active réticulée et qu'elle est simple à réaliser.

La présente invention concerne également l'utilisation des granules complexes pour la catalyse de réactions chimiques.

A cette fin, les granules sont disposés dans des réacteurs de diverses manières connues en elles-mêmes, sous forme de lit fixe, de lit fluidisé, ou encore de lit mobile ou de lit turbulent. Les granules complexes peuvent être complètement immergés dans le mélange réactionnel liquide, ou encore on peut faire ruisseler ce dernier sur les granules. Les procédés mettant en œuvre les

granules complexes peuvent être réalisés en continu ou en discontinu.

Ces procédés peuvent être appliqués avec succès dans les industries alimentaire et pharmaceutique. On peut ainsi les appliquer à la préparation d'acides aminés purs et éventuellement optiquement actifs tels que la méthionine, la lysine, la phénylalanine, le tryptophane et la valine. On peut également utiliser des granules complexes contenant de la glucose oxydase pour oxyder le glucose. De même, on peut utiliser des granules complexes contenant de la glucose isomérase pour isomérer le glucose en fructose. Dans ce dernier cas, le procédé convient bien à l'enrichissement en fructose de mélanges glucose-fructose.

La mise en œuvre des granules complexes selon l'invention pour effectuer des réactions chimiques est particulièrement intéressante. En effet, grâce à leur forme solide, on peut à tout moment arrêter la réaction en les retirant du mélange réactionnel. En outre, les produits de réactions ne sont pas contaminés. Enfin, les granules complexes conservent leur activité pendant des durées plus longues.

Les granules complexes présentent également une stabilité physique (résistance à l'attrition et à l'écrasement, et stabilité dimensionnelle) particulièrement grande. Ils peuvent donc être utilisés sans dommage dans tous les types de réacteurs.

En outre, étant donné que les substances protéiniques actives se retrouvent, non à la surface des granules, mais à l'intérieur des cavités, on n'observe quasi pas de pertes de substance active par attrition en cours d'utilisation et les granules n'ont pas tendance à s'agglomérer.

La présente invention concerne également un procédé pour la régénération du support des granules complexes après désactivation complète des substances actives. Le procédé convient particulièrement bien lorsque du titane spongieux est utilisé comme support.

Le procédé consiste à débarasser le support des granules complexes désactivés des substances qui y sont encore fixées et ensuite à traiter le support au moyen d'une solution aqueuse alcaline.

Le traitement au moyen d'une solution alcaline peut se faire selon diverses techniques connues en elles-mêmes telles que l'immersion du support dans la solution ou l'écoulement de la solution sur le support.

Divers types d'agents alcalins peuvent être utilisés. De bons résultats ont été obtenus avec l'hydroxyde de sodium. La solution aqueuse alcaline peut contenir des quantités variables d'alcali ; elles sont, en général comprises entre 0,01 et 10 moles, et, le plus souvent, entre 0,1 et 5 moles d'alcali par litre de solution.

Après le traitement alcalin, on peut éventuellement laver le support. Le support est ensuite séché et réutilisé.

Le séchage peut se faire de différentes manières connues en elles-mêmes. Une façon de faire consiste à chauffer le support à haute température, en général à des températures de 50 à 200 °C. On peut également soumettre le support à une ventilation par un gaz inerte (air, azote, argon). Le chauffage peut être poursuivi pendant des durées variables dépendant notamment de la température utilisée.

Sans le traitement au moyen d'une solution alcaline on constate qu'il est très difficile de réimprégner à nouveau le support débarassé des substances qui y étaient fixées.

Pour débarrasser le support désactivé des diverses substances qui y sont fixées, diverses techniques peuvent être utilisées. Une façon de faire adéquate consiste à soumettre le support désactivé à un traitement avec un oxydant, et plus particulièrement avec de l'hypochlorite de sodium, et ensuite, éventuellement, à laver le support ainsi traité.

Le traitement à l'hypochlorite de sodium peut se faire selon diverses techniques connues en elles-mêmes telles que l'immersion avec éventuellement fluidisation ou l'écoulement. Les solution aqueuses d'hypochlorite de sodium mises en œuvre contiennent en général de 1 à 50 % et le plus souvent de 5 à 40 % en poids d'hypochlorite de sodium. De bons résultats ont été obtenus avec des solutions à 20 % en poids d'hypochlorite de sodium. Ce traitement peut avoir des durées variables allant en général de 1 minute à 5 heures et le plus souvent de 2 minutes à 2 heures. Après le traitement à l'hypochlorite, la solution est éliminée du support selon toute technique connue en elle-même telle que l'égouttage par gravité, l'essorage et la filtration.

Le support peut être éventuellement soumis à un ou plusieurs rinçages à l'eau. Un rinçage peut être avantageusement réalisé au moyen d'une solution aqueuse contenant de petites quantités de sulfite de sodium, en général comprises entre 0,001 et 1 mole par litre de solution, et être suivi d'un rinçage à l'eau.

Le traitement de régénération permet de récupérer le suppport, et plus particulièrement le titane spongieux, sans perte et sans dégradation. Le support peut ainsi subir un très grand nombre d'imprégnations et de régénérations successives sans modification sensible.

Les exemples ci-après illustrent l'invention.

Exemple 1

Immobilisation de la glucose isomérase sur le titane spongieux

Produit A

On prépare une solution de glucose isomérase en dissolvant 31,5 g de protéines actives par litre d'une solution de pH 7,0 contenant 0,01 mol/l de tampon phosphate et 0,005 mol/l de Mg SO$_4$.

A 125 mg d'ovalbumine en poudre on ajoute successivement :

— 360 microlitre d'une solution de pH 6,88 contenant 0,02 mol/l de tampon phosphate ;

— 860 microlitre de la solution protéinique précitée, et

— 80 microlitre d'une solution contenant 0,2 mol/l de MgSO₄.

Après dissolution complète de l'albumine on ajoute 600 microlitre d'une solution de pH 6,88 contenant 75 mg/ml de glutaraldéhyde et 0,02 mol/l de tampon phosphate.

Après homogénéisation on ajoute 2 g de titane spongieux préalablement lavé. Le titane spongieux mis en œuvre est constitué de granulés ayant des dimensions comprises entre 0,5 et 1 mm, une surface spécifique de 0,130 m²/g, une porosité de 0,28 cm³/g, un diamètre moyen des cavités de 63 μ et une répartition des dimensions des cavités telle que environ 95 % du volume total des cavités est constitué de cavités ayant un diamètre compris entre 5 et 100 μm.

Le mélange ainsi obtenu est mis sous vide afin d'éliminer l'air occlus et après retour à la pression atmosphérique il est maintenu pendant au moins 4 h à − 35 °C.

Le produit est ensuite ramené à la température ambiante et lavé successivement avec une solution de glycocolle et une solution de tampon phosphate.

Dans les granules complexes obtenus, la concentration en ovalbumine est de 56,9 mg/g, en glucose isomérase de 12,3 mg/g, et en titane de 910 mg/g.

Produit B

On prépare une solution de glucose isomérase en dissolvant 26,3 g de protéines par litre d'une solution de pH 7,0 contenant 0,01 mol/l de tampon phosphate et 0,005 mol/l de MgSO₄.

A 125 mg d'ovalbumine en poudre on ajoute successivement :

— 600 microlitre d'une solution de pH 6,88 contenant 0,02 mol/l de tampon phosphate ;

— 1 ml de la solution protéinique précitée et

— 150 microlitre d'une solution de pH 6,88 contenant 250 mg/ml de glycérine et 0,02 mol/l de tampon phosphate.

Après dissolution complète de l'ovalbumine on ajoute 150 microlitre d'une solution de pH 6,88 contenant 75 mg/ml de glutaraldéhyde et 0,02 mol/l de tampon phosphate.

Après homogénéisation on ajoute 2 g de titane spongieux de même qualité que pour le produit A.

Le mélange est mis sous vide afin d'éliminer l'air occlus et après retour à la pression atmosphérique il est maintenu pendant 2 heures au moins dans une enceinte ventilée portée à 25-30 °C.

Le produit est ensuite lavé successivement avec une solution de glycocolle et une solution de tampon phosphate.

Dans les granules complexes obtenus la concentration en ovalbumine est de 57,8 mg/g, en glucose isomérase de 12,2 mg/g et en titane de 925 mg/g.

Isomérisation du glucose en fructose

Le catalyseur est disposé en lit fixe dans un réacteur tubulaire de 14 mm de diamètre intérieur. La température du réacteur est maintenue à 70 °C et on alimente en continu le réacteur à raison de 15-16 ml/h au moyen d'une solution aqueuse contenant · 2,4 mol/l de glucose, 0,02 mol/l de tampon sulfite, 0,005 mol/l de MgSO₄ et 0,75 g/l de p. hydroxybenzoate de méthyle. Ce dernier est utilisé pour éviter la formation et le développement de microorganismes. Le pH est égal à 7,5 et le temps de séjour est de 20 minutes.

Deux essais ont été réalisés avec respectivement le produit A (essai 1) et le produit B (essai 2). Dans les deux cas, on a observé un taux de conversion du glucose tel qu'il y a eu formation de 1 g de fructose par heure et par ml de catalyseur. Dans l'essai 1 la durée de demi vie du catalyseur fut de 15,5 jours et dans l'essai 2 de 16,5 jours.

Le même essai réalisé avec, comme catalyseur, une mousse constituée de glucose isomérase traitée comme indiqué pour le produit A mais sans introduction de titane a conduit au bouchage du réacteur après quelques heures de fonctionnement.

Exemple 2

Immobilisation de la glucose oxydase sur le titane spongieux

Produit C

On prépare une solution de glucose oxydase en dissolvant 11 g de protéines actives présentant une activité en catalase par litre d'une solution de pH 6,88 contenant 0,02 mol/l de tampon phosphate.

A 640 mg d'albumine bovine en poudre on ajoute successivement :

— 4 cm³ d'une solution de pH 6,88 contenant 0,02 mol/l de tampon phosphate, et

— 3,2 cm³ de la solution protéinique précitée.

Après dissolution complète de l'albumine, on ajoute 4 cm³ d'une solution de pH 6,88 contenant 15 g/l de glutaraldéhyde et.0,02 mol/l de tampon phosphate.

Après homogénéisation on ajoute 18 g de titane spongieux préalablement lavé. Le titane spongieux mis en œuvre est constitué de granulés ayant des dimensions comprises entre 1 et 2 mm, une surface spécifique de 0,130 m²/g, une porosité de 0,28 cm³/g, un diamètre moyen des cavités de 63 μm et une répartition des dimensions des cavités telle que environ 95 % du volume total des cavités est constitué de cavités ayant un diamètre compris entre 5 et 100 μm.

Le mélange ainsi obtenu est mis sous vide afin d'éliminer l'air occlus et après retour à la pression atmosphérique il est maintenu pendant au moins 4 h à − 35 °C.

Le produit est ensuite ramené à la température ambiante et lavé successivement avec une solution de glycocolle et une solution de tampon phosphate.

Dans les granules complexes òbtenus, la concentration en albumine est de 34 mg/g, en glucose oxydase de 1,87 mg/g, et en titane de 961 mg/g.

## Oxydation du glucose en acide gluconique

Le catalyseur est disposé en suspension dans 100 cm³ d'une solution à 40 g/l de glucose. La température est maintenue à 25 °C et le pH à 6,88 par addition continue d'une solution aqueuse 1N d'hydroxyde de sodium. On fait barboter au travers de la solution, à l'aide d'un verre fritté, de l'air épuré. Après 10 heures de fonctionnement on a transformé 25 g de glucose en acide gluconique.

## Exemple 3

Immobilisation de la glucoseisomérase sur du titane spongieux modifié par un échangeur d'ions

### Préparation du support

On dissout 8 g d'agarose dans 100 ml d'eau par chauffage pendant 10 min. à 120 °C dans le ballon de 500 ml. A la solution refroidie à 95 °C, on ajoute 140 g de « titane spongieux » de même qualité qu'à l'exemple 2. Le tout est chauffé pendant 10 min. à 120 °C et ensuite maintenu pendant 16 h à température ambiante. Au mélange, dont il reste 240 g et dont le volume apparent est de 200 ml, qui est dispersé dans le ballon de 1 l contenant 100 ml d'eau, on ajoute ensuite 200 ml de NaOH 1N, 4 ml d'épichlorohydrine et 1 g de borohydrure de sodium et on chauffe le tout sous agitation pendant 1 h à 60 °C. Les granules contenant l'agarose réticulée sont ensuite décantées et lavées à l'eau jusqu'à pH 7.

La totalité des granules réticulées est placée dans un ballon de 500 ml dans lequel on ajoute 33 g de chlorhydrate de 2-chloro-N, N-diéthylamine et 33 ml d'eau et le tout est agité pendant 30 min. à température ambiante. Ensuite on introduit 130 ml de NaOH 2,5 N et l'on continue l'agitation pendant 60 min. supplémentaires. Les granules sont décantées et lavées ave une solution de NaCl à 5 % jusqu'à pH 7 et ensuite avec de l'eau jusqu'à absence de chlorures dans l'effluent. Ce produit est conservé humide en présence d'un bactéricide.

### Fixation de la glucose isomérase

On dispose 1,8 ml du support préparé dans une colonne tubulaire de 14 mm de diamètre intérieur, et dans laquelle on fait circuler en cycle fermé 5 ml d'une solution de glucose isomérase contenant 12 mg de protéines par millilitre.

Après 3 h les protéines sont fixées sur le support.

### Isomérisation du glucose en fructose

On alimente en continu la colonne contenant les granules actives obtenues ci-avant et chauffée à 65 °C avec une solution aqueuse contenant 2,4 mol/l de glucose, 0,02 mol/l de tampon sulfite 0,005 mol/l de MgSO₄ et 0,5 g/l de p-hydroxybenzoate de méthyle comme antiseptique. Le pH est compris entre 7,2 et 7,4 et le débit est ajusté à une valeur voisine de 5 ml/h de façon à obtenir un taux de conversion de glucose en fructose de 42 %.

On constate qu'après 60 jours on a produit 2,3 kg de matière sèche et que le débit nécessaire pour maintenir un taux de conversion de 42 % est diminué à la moitié de sa valeur initiale.

Cet exemple de longue durée montre que les granules, contenant comme support un métal de l'invention, n'affectent pas les enzymes qui y sont fixés.

## Exemple 4

Rechargement en protéine active des granules

Des granules conformes à l'exemple 2 contenant les protéines actives dont l'activité est tombée à environ 25 % de la valeur initiale sont traités dans le réacteur qui les contient et après refroidissement, par une solution aqueuse de NaOH de 0,2-0,4 N. Ensuite on lave avec une solution de NaCl à 5 % jusqu'à pH 7 et ensuite avec de l'eau jusqu'à disparition des chlorures et puis on procède à la fixation des protéines actives comme décrit dans l'exemple 2.

Après sept cycles de régénération et d'utilisation en continu pendant 2 à 3 semaines à 70 °C, on n'observe aucune modification quant à la qualité du support, ni quant à la capacité en échange d'ions si l'on travaille selon l'exemple 2.

Ceci montre donc tout comme les exemples précédents, la qualité en tant que support inerte des métaux utilisés pour les granules selon l'invention.

## Revendications

1. Granules complexes contenant des substances protéiniques actives fixées sur un support solide minéral poreux insoluble dans l'eau constitué de granules caractérisé en ce que les granules du support comportent à l'intérieur de leur structure des cavités dont la majorité ont des dimensions moyennes comprises entre 5 et 200 µm.

2. Granules selon la revendication 1, caractérisés en ce que la dimension moyenne des cavités est comprise entre 20 et 100 µm.

3. Granules selon la revendication 1 ou 2, caractérisés en ce que leur surface spécifique est inférieure à 1 m²/g.

4. Granules selon l'une quelconque des revendications 1 à 3, caractérisés en ce que la solubilité dans l'eau du support poreux minéral est inférieure à 0,1 % en poids.

5. Granules selon l'une quelconque des revendications 1 à 4, caractérisés en ce que au moins 70 % du volume total des cavités est constitué de cavités ayant des dimensions comprises entre 5 et 200 μm.

6. Granules selon l'une quelconque des revendications 1 à 5, caractérisés en ce que le support minéral est constitué de titane spongieux.

7. Granules selon l'une quelconque des revendications 1 à 6, caractérisés en ce qu'ils contiennent des enzymes comme substances protéiniques actives.

8. Granules selon la revendication 7, caractérisés en ce que les enzymes sont choisies parmi la glucose oxydase, la glucose isomérase, la catalase, les lactases, la fumarase, les aminoacylases, la trypsine, la pepsine, les amylases, la pénicilline acylase, la pénicilline-amidase et les glycosyltransférases.

9. Granules selon l'une quelconque des revendications 1 à 8, caractérisés en ce qu'ils contiennent en outre des constituants polymériques inactifs choisis parmi les substances organiques d'origine animale ou végétale et les substances macromoléculaires de synthèse.

10. Granules selon la revendication 9, caractérisés en ce qu'ils contiennent des substances macromoléculaires de synthèse ayant des propriétés échangeuses d'ions.

11. Procédé pour la fabrication de granules selon l'une quelconque des revendications 1 à 10, caractérisé en ce qu'après imprégnation du support minéral par les substances protéiniques actives et les autres constituants éventuels de la composition on procède à une réticulation.

12. Procédé pour l'isomérisation du glucose en fructose caractérisé en ce qu'on utilise comme catalyseur des granules selon l'une quelconque des revendications 1 à 10 contenant du glucose isomérase.

13. Procédé pour l'oxydation du glucose en acide gluconique caractérisé en ce qu'on utilise comme catalyseur des granules selon l'une quelconque des revendications 1 à 10 contenant du glucose oxydase.

14. Procédé pour la régénération des granules selon l'une quelconque des revendications 1 à 10 caractérisé en ce qu'on débarasse les granules désactivés, des substances qui y sont fixées et qu'on traite ensuite le support au moyen d'une solution aqueuse alcaline avant de le remettre en œuvre pour fabriquer les granules.

## Claims

1. Complex granules containing active protein substances fixed to a water-insoluble, porous, solid mineral support consisting of granules, characterised in that the granules of the support possess cavities inside their structure, the majority of the cavities having average sizes of between 5 and 200 μm.

2. Granules according to Claim 1, characterised in that the average size of the cavities is between 20 and 100 μm.

3. Granules according to Claim 1 or 2, characterised in that their specific surface area is less than 1 m²/g.

4. Granules according to any one of Claims 1 to 3, characterised in that the solubility of the porous mineral support in water is less than 0.1 % by weight.

5. Granules according to any one of Claims 1 to 4, characterised in that at least 70 % of the total volume of the cavities consists of cavities having sizes of between 5 and 200 μm.

6. Granules according to any one of Claims 1 to 5, characterised in that the mineral support consists of titanium sponge.

7. Granules according to any one of Claims 1 to 6, characterised in that they contain enzymes as the active protein substances.

8. Granules according to Claim 7, characterised in that the enzymes are chosen from amongst glucose oxidase, glucose isomerase, catalase, lactases, fumarase, aminoacylases, trypsin, pepsin, amylases, penicillin acylase, penicillin amidase and glycosyltransferases.

9. Granules according to any one of Claims 1 to 8, characterised in that they also contain inactive polymeric constituents chosen from amongst organic substances of animal or vegetable origin and synthetic macromolecular substances.

10. Granules according to Claim 9, characterised in that they contain synthetic macromolecular substances having ion exchange properties.

11. Process for the manufacture of granules according to any one of Claims 1 to 10, characterised in that, after impregnation of the mineral support with the active protein substances and the other constituents, if any, of the composition, crosslinking is carried out.

12. Process for the isomerisation of glucose to fructose, characterised in that granules according to any one of Claims 1 to 10, containing glucose isomerase, are used as the catalyst.

13. Process for the oxidation of glucose to gluconic acid, characterised in that granules according to any one of Claims 1 to 10, containing glucose oxidase, are used as the catalyst.

14. Process for the regeneration of the granules according to any one of Claims 1 to 10, characterised in that the deactivated granules are freed of the substances which are fixed thereto, and in that the support is then treated by means of an alkaline aqueous solution before being re-used for manufacturing the granules.

## Ansprüche

1. Komplexe Körnchen, die aktive Proteinsubstanzen enthalten, welche auf einem festen, mineralischen, porösen, in Wasser unlöslichen Träger, bestehend aus Körnchen, fixiert sind, dadurch gekennzeichnet, dass die Körnchen des Trägers im Inneren ihrer Struktur Hohlräume aufweisen, von denen der überwiegende Anteil mittlere Abmessungen zwischen 5 und 200 μm besitzt.

2. Körnchen nach Anspruch 1, dadurch gekennzeichnet, dass die mittlere Grösse der Hohlräume zwischen 20 und 100 μm liegt.

3. Körnchen nach Anspruch 1 oder 2, dadurch gekennzeichnet, dass ihre spezifische Oberfläche unter 1 m²/g liegt.

4. Körnchen nach einem der Ansprüche 1 bis 3, dadurch gekennzeichnet, dass die Löslichkeit des porösen, mineralischen Trägers in Wasser unter 0,1 Gew.-% beträgt.

5. Körnchen nach einem der Ansprüche 1 bis 4, dadurch gekennzeichnet, dass wenigstens 70 % des Gesamtvolumens der Hohlräume aus Hohlräumen mit Abmessungen zwischen 5 und 200 μm bestehen.

6. Körnchen nach einem der Ansprüche 1 bis 5, dadurch gekennzeichnet, dass der mineralische Träger aus Titanschwamm besteht.

7. Körnchen nach einem der Ansprüche 1 bis 6, dadurch gekennzeichnet, dass die als aktive Proteinsubstanzen Enzyme enthalten.

8. Körnchen nach Anspruch 7, dadurch gekennzeichnet, dass die Enzyme ausgewählt sind unter Glucoseoxidase, Glucoseisomerase, Katalase, Lactasen, Fumarase, Aminoacylasen, Trypsin, Pepsin, Amylasen, Penicillinacylase, Penicillinamidase und Glycosyltransferasen.

9. Körnchen nach einem der Ansprüche 1 bis 8, dadurch gekennzeichnet, dass sie zusätzlich inaktive polymere Bestandteile, ausgewählt unter organischen Substanzen tierischen oder pflanzlichen Ursprungs und synthetischen makromolekularen Substanzen, enthalten.

10. Körnchen nach Anspruch 9, dadurch gekennzeichnet, dass sie synthetische makromolekulare Substanzen mit Ionenaustauschereigenschaften enthalten.

11. Verfahren zur Herstellung der Körnchen nach einem der Ansprüche 1 bis 10, dadurch gekennzeichnet, dass man nach einer Imprägnierung des mineralischen Trägers mit den aktiven Proteinsubstanzen und den eventuellen weiteren Bestandteilen der Zusammensetzung eine Vernetzung vornimmt.

12. Verfahren zur Isomerisation von Glucose zu Fructose, dadurch gekennzeichnet, dass man als Katalysator Körnchen nach einem der Ansprüche 1 bis 10 mit einem Gehalt an Glucoseisomerase verwendet.

13. Verfahren zur Oxidation von Glucose zu Gluconsäure, dadurch gekennzeichnet, dass man als Katalysator Körnchen nach einem der Ansprüche 1 bis 10 mit einem Gehalt an Glucoseoxidase verwendet.

14. Verfahren zur Regeneration von Körnchen nach einem der Ansprüche 1 bis 10, dadurch gekennzeichnet, dass man die desaktivierten Körnchen von den daran fixierten Substanzen befreit und hierauf den Träger mit einer wässerigen alkalischen Lösung behandelt, bevor man ihn wieder zur Herstellung der Körnchen einsetzt.